# EUROPÄISCHE PATENTSCHRIFT

(11) **EP 1 108 715 B1**
(45) Veröffentlichungstag und Bekanntmachung des Hinweises auf die Patenterteilung: **08.11.2006**
(21) Anmeldenummer: 00124368.2
(22) Anmeldetag: 20.11.2000
(51) Int. Cl.: C07D 295/02

(54) **Verfahren zur Entwässerung und Reinigung von Rohpyrrolidin**
Process for dehydration and purification of crude pyrrolidine
Procede de deshydratation et purification de la pyrrolidine brute

(30) Priorität: 30.11.1999 DE 19957672
(43) Veröffentlichungstag der Anmeldung: 20.06.2001
(73) Patentinhaber: BASF Aktiengesellschaft, 67056 Ludwigshafen (DE)
(72) Erfinder: Simon, Joachim, Dr., 68161 Mannheim (DE); Wahl, Peter, Dr., 68526 Ladenburg (DE); Schmidt, Willi, Dr., 67069 Ludwigshafen (DE)
(74) Vertreter: Isenbruck, Günter

(56) Entgegenhaltungen:
- DE-C- 701 825
- US-A- 2 525 584
- DATABASE WWW.SIGMA-ALDRICH.COM [Online] Aldrich-Chimie S.a.r.l.; Pr-Nr.:394238, 1990 XP002160551

## Beschreibung

Die Erfindung betrifft ein Verfahren zur Entwässerung und Reinigung von Rohpyrrolidin. Verfahrensprodukt ist wasserfreies Pyrrolidin, d.h. mit einem Wassergehalt von weniger als 0,3% und einem Pyrrolidingehalt von mehr als 99% (Prozentangaben beziehen sich immer auf das Gewicht).

Wasserfreies Pyrrolidin ist ein wertvolles Zwischenprodukt und findet z.B. bei der Synthese von Pflanzenschutzmitteln und Pharmazeutika Anwendung.

Pyrrolidin wird heute überwiegend durch die Umsetzung von Butandiol mit Ammoniak unter Verwendung eines Nickel/Kupfer-Katalysators hergestellt. Ein solches Verfahren ist in EP 0 070 397 A1 beschrieben. Das nach diesem Verfahren hergestellte Rohpyrrolidin enthält neben Spuren niedrigsiedender Verunreinigungen wie Tetrahydroforan (THF) noch Hochsieder wie N-Butylpyrrolidin, N-(4-Aminobutyl)-pyrrolidin und 1,4-Bis-(N-pyrrolidino)-butan und 40% Wasser, das bei der Reaktion gemäß der allgemeinen Formel [1] gebildet wird: 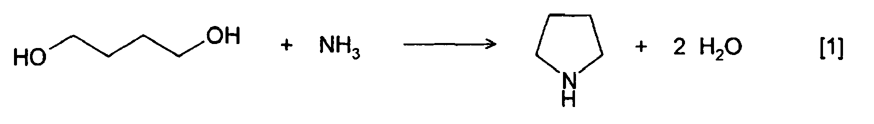 Obwohl Pyrrolidin mit Wasser bei Normaldruck kein Azeotrop bildet, hat sich die Abtrennung von Wasser als problematisch erwiesen.

Bisher wurde dem Rohpyrrolidin soviel 50%-ige Natronlauge zugesetzt, daß die Lauge nachher eine Konzentration von 25% aufweist. Nach intensiver Durchmischung wurde die obere organische Phase abgetrennt. Diese Phase enthält neben Pyrrolidin noch 3 bis 5% Wasser, was bisher durch Schleppmitteldestillation mit Hexan als Schleppmittel entfernt wurde. Das so erhaltene wasserfreie Pyrrolidin wurde anschließend fraktioniert destilliert, wobei reines Pyrrolidin mit einem Wassergehalt von unter 0,3% erhalten wurde. Die Entwässerung mit Hilfe der Schleppmitteldestillation ist außerordentlich zeit- und damit auch kostenintensiv. Außerdem beinhaltet das bekannte Verfahren drei Verfahrensschritte, wobei zwei auf getrennte Destillationsvorgänge entfallen, was einen erhöhten apparativen Aufwand und entsprechende Ausbeuteverluste nach sich zieht.

Es ist somit Aufgabe der vorliegenden Erfindung, ein Verfahren zur Entwässerung und Reinigung von Rohpyrrolidin anzugeben, welches in einem Destillationsschritt wasserfreies und spezifikationsgerechtes Pyrrolidin (Wasser max. 0,3%, Pyrrolidin min. 99%) erzeugt.

Die Erfindung geht aus von einem Verfahren zur Reinigung von Rohpyrrolidin unter Erhalt einer Pyrrolidinzusammensetzung von mehr als 99 % (m/m) Pyrrolidin und weniger als 0,3 % (m/m) Wasser.

Das erfindungsgemäße Verfahren ist dann dadurch gekennzeichnet, daß Rohpyrrolidin, enthaltend 40 bis 98 % (m/m) Pyrrolidin, mit einem Wassergehalt von mehr als 2 % (m/m) zur kontinuierlichen Destillation bei einem Kopfdruck von weniger als 950 hPa einer Kolonne mit Abtriebsteil und Verstärkerteil zugeführt wird.

Vorzugsweise wird im Rahmen des erfindungsgemäßen Verfahrens Rohpyrrolidin verwandt, das durch eine Reaktion gemäß der vorstehenden allgemeinen Formel [1] hergestellt wurde.
Die Anwendung des erfindungsgemäßen Verfahrens ermöglicht sowohl die Einsparung einer Destillationsstufe als auch die Einsparung der Entwässerung des Rohpyrrolidins mit Natronlauge. Ein vorgeschalteter Entwässerungsschritt mit Natronlauge ist erfindungsgemäß zwar möglich, aber überflüssig. Eine diskontinuierliche Destillation unter vermindertem Druck ist zwar ebenfalls möglich. Jedoch ist in diesem Fall stets ein vorgeschalteter Entwässerungsschritt notwendig, um spezifikationsgerechtes Pyrrolidin zu erhalten.
Ein weiterer Vorteil des erfindungsgemäßen Verfahrens ist die hohe Pyrrolidinausbeute von mindestens 90 % bezogen auf eingesetztes Rohpyrrolidin, die bisher nur mit mehrstufigem Verfahren erzielbar war. Schließlich weist das erfindungsgemäße Verfahren alle Vorteile eines kontinuierlichen Verfahrens auf, wie gleichbleibende Produktqualität und geringen Wartungsaufwand. Dies vereinfacht die Qualitätskontrolle erheblich.

In einer weiteren Ausführungsform des erfindungsgemäßen Verfahrens wird ein Kopfdruck von weniger als 500 hPa, vorzugsweise von 200 bis 350 hPa, insbesondere von 250 bis 350 hPa angelegt.

Eine im Rahmen der Erfindung einsetzbare Kolonne weist bevorzugt etwa 30 bis 40 theoretische Böden auf, wovon bevorzugt dieselbe Anzahl von theoretischen Böden auf Verstärker- und Abtriebsteil entfällt.

In einer weiteren Ausführungsform des erfindungsgemäßen Verfahrens wird ein Rücklaufverhältnis von 3:1 bis 10:1 (vorzugsweise von 4:1 bis 6:1) eingestellt. Das zu wählende Rücklaufverhältnis hängt mit der Anzahl der theoretischen Böden der Kolonne zusammen und beträgt bei 32 theoretischen Böden vorzugsweise 5:1. Eine solche Kolonne wird bevorzugt bei 300 hPa betrieben. Die Erfindung wird näher durch das nachfolgende Beispiel beschrieben.

### Beispiel 1

Rohpyrrolidin der Zusammensetzung nach Tabelle 1 wird kontinuierlich in eine 32 theoretische Böden aufweisende Kolonne eingespeist, wovon jeweils 16 theoretische Böden auf Abtriebs- und Verstärkerteil entfallen.

**Tabelle 1**

| **Komponenten** | **% (m/m) bestimmt durch GC** | **g/h bestimmt durch GC** |
|---|---|---|
| Vorlauf | 0,15 | 0,5 |
| Pyrrolidin | 48,19 | 168,7 |
| Zwischenlauf | 0,05 | 0,2 |
| Aminobutylpyrrolidin | 0,68 | 2,4 |
| Dipyrrolidinobutan | 7,84 | 27,4 |
| Hydroxybutylpyrrolidin | 0,57 | 2,0 |
| Nachlauf | 3,52 | 12,3 |
| Wasser | 39,00 | 136,5 |

Es wurde ein Rücklaufverhältnis von 5:1 eingestellt. An Kopf- und Sumpfabzug herrschte eine Temperatur von 54 bis 55° bzw. 71 bis 73°. Dabei wurde dem Kopfabzug mit einer Rate von 160 g/h eine Flüssigkeit der Zusammensetzung von Tabelle 2 entnommen.

**Tabelle 2**

| **Komponenten** | **% (m/m)** **bestimmt durch GC** | **g/h** **bestimmt durch GC** |
|---|---|---|
| Vorlauf | 0,44 | 0,7 |
| Pyrrolidin | 99,39 | 159,1 |
| Zwischenlauf | 0,02 | -- |
| Aminobutylpyrrolidin | -- | -- |
| Dipyrrolidinobutan | -- | -- |
| Hydroxybutylpyrrolidin | -- | -- |
| Nachlauf | -- | -- |
| Wasser | 0,15 | 0,2 |

| | | |
|---|---|---|
| Vorlauf: Summe der im Gaschromatogramm sichtbaren Komponenten mit kleineren Retentionszeiten als Pyrrolidin. Zwischenlauf: Summe der im Gaschromatogramm sichtbaren Komponenten mit Retentionszeiten zwischen denen des Pyrrolidins und des Aminobutylpyrrolidins Nachlauf: Summe der im Gaschromatogramm sichtbaren Komponenten mit höheren Retentionszeiten als Hydroxybutylpyrrolidin. | | |

Dem Sumpfabzug wurde mit einer Rate von 190 g/h eine Flüssigkeit gemäß Tabelle 3 entnommen.

**Tabelle 3**

| **Komponenten** | **% (m/m)** **bestimmt durch GC** | **g/h** **bestimmt durch GC** |
|---|---|---|
| Vorlauf | -- | -- |
| Pyrrolidin | 5,45 | 10,4 |
| Zwischenlauf | 0,12 | 0,2 |
| Aminobutylpyrrolidin | 1,42 | 2,7 |
| Dipyrrolidinobutan | 14,76 | 28,0 |
| Hydroxybutylpyrrolidin | 1,25 | 2,4 |
| Nachlauf | 8,9 | 16,9 |
| Wasser | 68,10 | 129,4 |

Die Destillationsausbeute betrug 94%.

## Patentansprüche

1. Verfahren zur Reinigung von Rohpyrrolidin unter Erhalt einer Pyrrolidinzusammensetzung von mehr als 99 % (m/m) Pyrrolidin und weniger als 0,3 % (m/m) Wasser, **dadurch gekennzeichnet, daß** Rohpyrrolidin, enthaltend 40 bis 98 % (m/m) Pyrrolidin, mit einem Wassergehalt von mehr als 2 % (m/m) zur kontinuierlichen Destillation bei einem Kopfdruck von weniger als 950 hPa einer Kolonne mit Abtriebsteil und Verstärkerteil zugeführt wird.

2. Verfahren nach Anspruch 1, **dadurch gekennzeichnet, daß** ein Kopfdruck von weniger als 500 hPa angelegt wird.

3. Verfahren nach Anspruch 2, **dadurch gekennzeichnet, daß** ein Kopfdruck von 200 bis 400 hPa angelegt wird.

4. Verfahren nach Anspruch 3, **dadurch gekennzeichnet, daß** ein Kopfdruck von 250 bis 350 hPa angelegt wird.

5. Verfahren nach einem der Ansprüche 1 bis 4, **dadurch gekennzeichnet, daß** der Ort der Einspeisungen des zu trennenden Gemisches so gewählt wird, daß dieselbe Anzahl von theoretischen Böden auf Abtriebsteil und Verstärkerteil entfällt.

6. Verfahren nach Anspruch 1, **dadurch gekennzeichnet, daß** ein Rücklaufverhältnis von 3:1 bis 10:1 eingestellt wird.

## Claims

1. A process for purifying crude pyrrolidine to give a pyrrolidine composition comprising more than 99% (m/m) of pyrrolidine and less than 0.3% (m/m) of water, wherein crude pyrrolidine comprising from 40 to 98% (m/m) of pyrrolidine and having a water content of more than 2% (m/m) is subjected to continuous distillation at a pressure at the top of less than 950 hPa in a column having a stripping section and a rectifying section.

2. The process according to claim 1, wherein a pressure at the top of less than 500 hPa is employed.

3. The process according to claim 2, wherein a pressure at the top of from 200 to 400 hPa is employed.

4. The process according to claim 3, wherein a pressure at the top of from 250 to 350 hPa is employed.

5. The process according to any of claims 1 to 4, wherein the place at which the mixture to be fractionated is fed in is selected so that the number of theoretical plates in the stripping section is the same as that in the rectifying section.

6. The process according to claim 1, wherein a reflux ratio of from 3:1 to 10:1 is set.

## Revendications

1. Procédé de purification de pyrrolidine brute en vue d'obtenir une composition de pyrrolidine qui contient plus de 99 % (moles/moles) de pyrrolidine et moins de 0,3 % (moles/moles) d'eau, **caractérisé en ce que** la pyrrolidine brute qui contient de 40 à 98 % (moles/moles) de pyrrolidine et dont la teneur en eau est supérieure à 2 % (moles/moles) est amenée à une distillation en continu à une pression de tête inférieure à 950 hPa dans une colonne qui présente une partie de rectification et une partie d'amplification.

2. Procédé selon la revendication 1, **caractérisé en ce que** l'on applique une pression de tête inférieure à 500 hPa.

3. Procédé selon la revendication 2, **caractérisé en ce que** l'on applique une pression de tête comprise entre 200 et 400 hPa.

4. Procédé selon la revendication 3, **caractérisé en ce qu'**on applique une pression de tête comprise entre 250 et 350 hPa.

5. Procédé selon l'une des revendications 1 à 4, **caractérisé en ce que** l'emplacement d'injection du mélange à séparer est sélectionné de telle sorte que l'on obtienne un même nombre de fonds théoriques dans la partie de rectification et dans la partie d'amplification.

6. Procédé selon la revendication 1, **caractérisé en ce que** l'on y ajuste un rapport de recirculation de 3 : 1 à 10 : 1.
